# EUROPÄISCHE PATENTSCHRIFT

(11) **EP 0 739 199 B1**
(45) Veröffentlichungstag und Bekanntmachung des Hinweises auf die Patenterteilung: **03.04.2002**
(21) Anmeldenummer: 95906300.9
(22) Anmeldetag: 05.01.1995
(51) Int. Cl.: A61K 9/70

(54) **ESTRADIOL-PENETRATIONSVERSTÄRKER ZUR TRANSDERMALEN VERABREICHUNG**
ESTRADIOL PENETRATION INTENSIFIER FOR TRANSDERMAL ADMINISTRATION
AMPLIFICATEUR DE PENETRATION D'ESTRADIOL POUR ADMINISTRATION TRANSCUTANEE

(30) Priorität: 13.01.1994 DE 4400770
(43) Veröffentlichungstag der Anmeldung: 30.10.1996
(73) Patentinhaber: LTS LOHMANN Therapie-Systeme AG, 56626 Andernach (DE)
(72) Erfinder: MECONI, Reinhold, D-56567 Neuwied (DE); SEIBERTZ, Frank, D-53557 Bag Hönningen/Ariendorf (DE)
(74) Vertreter: Flaccus, Rolf-Dieter, Dr.
(86) Internationale Anmeldenummer: EP9500032
(87) Internationale Veröffentlichungsnummer: WO9519162

(56) Entgegenhaltungen:
- EP-A- 0 364 211
- EP-A- 0 483 370
- WO-A-92/07589
- DE-A- 4 223 360
- US-A- 4 732 892
- DATABASE WPI Section Ch, Week 9419 Derwent Publications Ltd., London, GB; Class A14, AN 94-156550 & JP,A,06 100 439 (SEKISUI CHEM. IND. CO. LTD) , 12.April 1994

## Beschreibung

Die Erfindung betrifft ein wirkstoffhaltiges Pflaster zur kontrollierten Abgabe von Estradiol oder seinen pharmazeutisch unbedenklichen Derivaten alleine oder in Kombination mit Gestagenen an die menschliche oder tierische Haut unter Verwendung von Haftklebern und mindestens einem Penetrationsverstärker, seine Verwendung und ein Verfahren zu seiner Herstellung.

Oestrogenhaltige Pflaster sind bereits bekannt. Sie weisen jedoch die Nachteile auf, daß sie entweder Ethanol enthalten oder die potentielle Gefahr besitzen, daß der Wirkstoff im Laufe der Zeit rekristallisiert oder daß sie Estradiol nicht in einer für eine Therapie ausreichenden Menge freisetzen.

Aus der DE OS 32 05 258 und der EP 0 285 563 ist bekannt, Estradiol und Ethanol gleichzeitig in einer Pflasterformulierung zu verabreichen. Die Herstellung dieses Pflasters ist jedoch sehr aufwendig und der Tragekomfort nach Applikation aufgrund der fehlenden Flexibilität gering.

Die EP-A-0 285 563 beschreibt ein transdermales therapeutisches System für die kombinierte Applikation von Oestrogenen und Gestagenen. Das Reservoir enthält die Wirkstoffformulierung, und C₂-C₄-Alkanol, insbesondere Ethanol, als perkutanes absorptionsverbesserndes Mittel. Die Freisetzung des Wirkstoffes wird hauptsächlich von einer Membran gesteuert. Eine Klebschicht hat die Funktion, das Pflaster auf der Haut zu befestigen. Es handelt sich hierbei vornehmlich um ein sogenanntes "Beutelpflaster", da sich die Wirkstoffzubereitung in einem Beutel, bestehend aus undurchlässiger Rückschicht und Membran mit Kleberschicht befindet. Infolge seines komplizierten Aufbaues ist die Herstellung des Pflasters sehr aufwendig, da die Einzelkomponenten separat hergestellt und dann in einem weiteren Arbeitsgang zu einem Pflaster zusammengefügt werden müssen.

Die EP 0 275 716 beschreibt ein zweischichtiges transdermales System zur simultanen Verabreichung von einem oder mehreren Oestrogenen, die in der Polymerschicht gelöst oder mikrodispergiert sind. Die Haftschicht enthält dabei außer den Wirkstoffen Substanzen, die die transdermale Absorption verbessern. Polymer- und Haftschicht können aus Polyacrylaten, Silikonen oder Polyisobutylenen bestehen.

In der EP 0 072 251 ist eine flexible, flüssigkeitsabsorbierende, medikamentöse Bandage beschrieben. Das an der flexiblen Rückschicht befestigte Substrat besteht aus einer hydrophilen Matrix auf der Basis von hydrophilen hochmolekularen Polysacchariden und/oder Polyacrylsäure, Polyacrylamid, Ethylen-Vinylacetat-Copolymeren und anderen Polymeren sowie einer flüssigen Phase auf der Basis einer Lösung oder Emulsion aus Kohlehydrat, Proteinen und mehrwertigen Alkoholen sowie verschiedenen Wirkstoffen, u.a. auch Hormonen. Wesentliches Merkmal dieser Erfindung ist der feuchtigkeitsabsorbierende Kleber.

Die EP 0 328 806 beschreibt ein membranfreies, transdermales therapeutisches System, dessen Matrix aus einem Polyacrylatkleber, einem Lösemittel, einem Polyoxyethylenester als Penetrationsverbesserer und Oestrogen, dessen Derivaten und Kombinationen davon besteht.
In der WO 87/07 138 ist ein Estradiol-Pflaster auf der Basis einer Rückschicht, einer den Wirkstoff enthaltenden Matrix und einem Haftkleber, der mit einer wiederablösbaren Schutzschicht abgedeckt ist, beschrieben. Die Herstellung von Matrix und Haftkleber erfolgen in technologisch sehr aufwendigen Arbeitsvorgängen durch Homogenisieren, Entgasen, Beschichten, Trocknen und Vereinzeln. In einer Ausführungsform muß die Rückschicht sogar mit einem Haftkleber beschichtet werden, was einen weiteren Arbeitsgang bedingt. Das Zusammenfügen der einzelnen Teile erfolgt in einem separaten Arbeitsgang. Die Herstellung des Pflasters ist also insgesamt sehr aufwendig und kompliziert.

Aus der US-PS 4 624 665 sind Systeme bekannt, die im Reservoir den Wirkstoff in mikroverkapselter Form enthalten. Das Reservoir ist eingebettet zwischen Rückschicht und einer Membran. Der äußere Rand des Systems ist mit einem Haftkleber ausgerüstet. Der Aufbau und die Herstellung dieses Systems ist sehr kompliziert, da der Wirkstoff mikroverkapselt und in einer flüssigen Phase homogen verteilt werden muß, die dann in weiteren Arbeitsgängen zwischen Rückschicht und Membran eingebettet wird. Zusätzlich muß das System dann mit einem klebenden Rand versehen und mit einer Schutzschicht abgedeckt werden.

Es sind weiterhin aus der EP 0 186 019 Wirkstoffpflaster bekannt, bei denen einer Kautschuk-Klebharzmasse in Wasser quellbare Polymere zugesetzt sind und aus denen Estradiol freigesetzt werden kann. Es hat sich jedoch gezeigt, daß die Freisetzung von Estradiol aus diesen Wirkstoffpflastern viel zu gering ist und nicht den therapeutischen Erfordernissen entspricht.

In der DE-OS 20 06 969 ist ein Pflaster oder ein Haftverband mit Systemwirkung beschrieben, bei dem empfängnisverhütende Substanzen in die Klebstoffkomponente oder den Klebstoffilm eingearbeitet wird. Der Klebstoffilm kann aus einem Acrylat bestehen.

Die DE-OS 39 33 460 beschreibt ein oestrogenhaltiges Wirkstoffpflaster auf der Basis von Homo- und/oder Copolymeren mit mindestens einem Derivat der Acryl- oder mit Methacrylsäure in Kombination mit in Wasser quellbaren Substanzen.

In der EP 0 430 491 ist ein transdermales therapeutisches System beschrieben, das die Penetration von Estradiol verstärkende Bestandteile enthält. Dazu gehören ungesättigte Fettsäuren, deren Alkylester und Glycerin bzw. Alkandiole, wie z.B. Propandiol. Diese Formulierung hat jedoch die Nachteile, daß die ungesättigten Fettsäuren oxidationsempfindlich sind und somit einer chemischen Veränderung unterliegen und außerdem Propandiol während des Trocknungsvorganges unkontrolliert verdampft und deshalb ein wirkstoffhaltiges Pflaster mit der geforderten konstanten Zusammensetzung nicht herzustellen ist.

Auch das in der EP 0 371 496 beschriebene transdermale System besitzt den Nachteil, daß es als Penetrationsverstärker Ölsäure enthält, die oxidationsempfindlich ist und auf diese Weise kein stabiles System herzustellen erlaubt, dessen Eigenschaften sich während der Lagerung nicht verändern.

Die EP 0 569 338 beschreibt ein Pflaster für die transdermale Verabreichung von Estradiol unter Verwendung von Penetrationsverstärkern. Dazu gehören gesättigte und ungesättigte Fettsäuren sowie Propylenglycol. Die ungesättigten Fettsäuren haben den Nachteil, daß sie oxidationsempfindlich sind und Propylenglycol während des Trocknungsvorganges in unkontrollierter Weise verdampft. Deshalb ist ein estradiolhaltiges Pflaster mit der erforderlichen konstanten Zusammensetzung, die sich auch während der Lagerung nicht verändert, nicht herzustellen.

Eine spezielle, Estradiol oder eine Ester enthaltende Klebschicht aus einem Copolymeren von 45 bis 80 mol% 2-Ethylhexyacrylat und 20 bis 55 mol% N-Vinyl-2-pyrolidon mit flexibler, wirkstoffundurchlässiger Rückschicht wird in der EP-A-0 364 211 beschreiben, die als Penetrationsverstärker Ester höhere Fettsäuren, Verbindungen mit Amidbindung bzw. Dicarbonsäuren 2 bis 10 C-Atomen enthalten kann. Als besondere Penetrationsverstäker werden Milchsäure, Zitronensäure, Fumarsäure und O-Alkyl-(polyoxyethyl)-phosphat genannt. Der relativ hohe Wirkstoffgehalt von 6 bis 25 Gew.% der wirkstoffhaltigen Klebmasse weist auf Freisetzungsschwierigkeiten hin, denen durch einen relativ hohne Wirkstoffvorrat begegnet werden soll.

Die WO 92/07589 beschreibt schließlich eine Zusammensetzung zur transdermalen Verabreichung von kontrazeptivem Wirkstoff zusammen mit Östrogen, insbesondere Ethinylestradiol, das bzw. die in einer Matrix mit permeationsverstärker enthalten sind. Als bevorzugte Substanzen für eine solche Permeationsverstärkung neben Alkohol als Enhancer werden insbesondere Monoglyceride von Fettsäuren, speziell Glycerinmonooleat, linoleat und lanrat genannt.

Insgesamt hat sich gezeigt, daß haftklebende transdermale therapeutische Matrix-Systeme, die den Wirkstoff teilweise oder vollständig gelöst enthalten, Estradiol nicht in der Menge freisetzen, die für eine Therapie erforderlich ist. Es ist versucht worden, diesen Nachteil dadurch zu beheben, daß man die Fläche der Wirkstoffpflaster vergrößert hat. Das hat jedoch die Konsequenz, daß sich die Pflaster während der Applikationszeit teilweise ablösen. Dadurch ist der für die Therapie erforderliche vollflächige Kontakt zur Haut nicht mehr gewährleistet und die durch die Haut penetrierende Wirkstoffmenge schwankt in unzulässiger Weise. Eine Therapie mit einer konstanten Wirkstoffgabe ist so nicht zu gewährleisten.

Es ist somit Aufgabe der Erfindung, die oben angeführten Nachteile zu vermeiden und ein oestrogenhaltiges Pflaster zur Verfügung zu stellen, das den Wirkstoff in ausreichender Menge freisetzt und den Nachteil einer unakzeptablen Pflastergröße und von Haftproblemen vermeidet.

Überraschenderweise hat sich gezeigt, daß die Aufgabe gelöst wird durch ein wirkstoffhaltiges Pflaster zur kontrollierten Abgabe von Estradiol oder seinen pharmazeutisch unbedenklichen Derivaten allein oder in Kombination mit Gestagenen, bestehend aus einer Rückschicht, einem damit verbundenen wirkstoffhaltigen Reservoir, das unter Verwendung von Klebharz enthaltenden Haftklebern hergestellt ist, und einer wiederablösbaren Schutzschicht, wobei der Haftkleber mindestens einen Penetrationsverstärker aus der Gruppe von Substanzen auf Basis spezieller Carbonsäuren insbesondere mit Hydroxy-, Amino- bzw. Keto-Funktion oder Säureamide enthält.

Diese Substanzen auf der Basis von Carbonsäuren sind ausgewählt aus der Gruppe Glycolsäure, Äpfelsäure, Weinsäure, Mandelsäure, 2-Hydroxyzimtsäure, 3-Hydroxyzimtsäure, trans-4-Methoxyzimtsäure, 2-Hydroxyoctansäure, Tropasäure, Gallussäure, Shikimisäure, Benzilsäure, Benzol-1,2,4 tricarbonsäure, Dimethyl-3-oxoglutarat, 3-Methyl-2-oxo-valeriansäure, 4-Methyl-2-oxo-valeriansäure, 2-Oxoglutarsäure, Brenztraubensäure, 4-Aminobuttersäure, 6-Aminohexansäure, 11 Aminoundecansäure, Asparaginsäure, 2-Aminobenzoesäure, 3- Aminobenzoesäure, 4-Aminobenzoesäure, 4-Amino-2 hydroxybenzoesäure, 3-Phenylpropionsäure, 2-Phenylbuttersäure, 4 Phenylbuttersäure, trans-2-Dodecendisäure, Tridecandisäure, Tetradecandisäure, Pentadecandisäure, Diglycolsäure, Piperidin-4-carbonsäure, Pyrazin-2-carbonsäure, Pyrazin-2,3-dicarbonsäure, Pyridin-2-carbonsäure und Nicotinsäure, Pimelinsäuremonomethylester, Malonsäurediamid, Adipinsäurediamid, Bernsteinsäurediamid, Pyrazin-2-carbonsäureamid.

Der Anteil an Penetrationsverstärkern auf Basis von Carbonsäuren beträgt 0,01 - 20 Gew.-%.

Der estradiolhaltige Haftkleber enthält Polymere, die ausgewählt sind aus der Gruppe der Styrol-Butadien-Styrol-Blockcopolymere, Styrol-Isopren-Styrol-Blockcopolymere, Styrol-Ethylen-Butylen-Styrol-Blockcopolymere, Polyisobutylene, Ethylen-Vinylacetat-Copolymere, Polyvinylpyrrolidon, Cellulosederivate, Polycaprolactame, Polycaprolactone, Ethylen-Ethylacrylat-Copolymer, Polyvinylether, Polyvinylacetale, Polyvinylacetate, Butyl-Kautschuke, Acrylnitril-Butadien-Copolymere, Polyethylenglycole und Polymere auf der Basis von Acrylsäure- und Methacrylsäurederivaten. Diese Polymere sind in einer Konzentration von mindestens 6 Gew.-% in der estradiolhaltigen Klebmasse enthalten.

Der Haftkleber enthält ferner klebrigmachende Harze in einer Konzentration von 5 - 94 Gew.-%. Diese sind dem Fachmann bekannt und in der US 5 126 144 beschrieben.

Das Wirkstoffpflaster enthält im Reservoir Estradiol oder seine pharmazeutisch unbedenklichen Derivate allein oder in Kombination mit Gestagenen in einer Konzentration von insgesamt 2 - 15 Gew.-%, und zwar in einem molaren Verhältnis von Estradiol oder seinen pharmazeutisch unbedenklichen Derivaten zu Gestagenen von 1 : 1 bis 1 : 10.

Das estradiolhaltige Reservoir kann mindestens einen Hilfsstoff aus der Gruppe enthalten, welche Farbstoffe, Füllstoffe, Alterungsschutzmittel, Weichmacher und Antioxidantien umfaßt. Diese Hilfsstoffe sind dem Fachmann bekannt und beispielsweise in der DE 37 43 946 beschrieben. Das estradiolhaltige Reservoir enthält üblicherweise Hilfsstoffe in einem Anteil von bis zu 5 Gew.-%.

Das Wirkstoffpflaster kann aus einer einzigen oder mehreren Schichten bestehen. Die Dicke des wirkstoffhaltigen Reservoirs kann 0,02 - 1,0 mm betragen.

Die Materialien für die undurchlässige Rückschicht und die wiederablösbare Schutzschicht sind dem Fachmann ebenso bekannt (z.B. DE 38 43 239).

Das estradiolhaltige Reservoir kann aus Lösung, aus Dispersion oder auch aus der Schmelze erzeugt werden.

Ferner kann das Reservoir aus mehreren Schichten bestehen.

Für den Fall, daß das Reservoir keine ausreichende Eigenklebrigkeit zur Haut aufweist, kann dieses mit einer Haftkleberschicht oder mit einem haftklebenden Rand versehen werden. Auf diese Weise ist gewährleistet, daß das transdermale Pflaster über den gesamten Applikationszeitraum auf der Haut haftet.

Ein besonders bevorzugter Aufbau des transdermalen estradiolhaltigen Pflasters ist das Matrixsystem, bei dem bekanntlich die Matrix die Steuerung für die Wirkstofffreisetzung übernimmt und diese dem √t-Gesetz nach Higuchi gehorcht. Das bedeutet jedoch nicht, daß nicht in besonderen Fällen auch das Membran-System angezeigt ist. Hierbei ist zwischen Reservoir und Haftkleberschicht eine die Wirkstofffreisetzung steuernde Membran angebracht.

Die Erfindung wird durch die folgenden Beispiele erläutert.

### Beispiel 1:

| | |
|---|---|
| 66,7 g | Triethylenglycolester von hydrierten Kolophonium (Staybelite® Ester 3E/Fa. Hercules) |
| 8,9 g | Glycerinester von hydriertem Kolophonium (Staybelite® Ester 10 E/Fa. Hercules), |
| 8,9 g | Ethylcellulose und |
| 1 g | Buthylhydroxianisol werden bei 165 °C ca. 1 1/2 Stunden durch Rühren homogenisiert. Anschließend werden |
| 10,0 g | DL-Äpfelsäure zugegeben und ca. 2 Stunden gerührt. Danach werden |
| 2,5 g | Estradiol zugegeben und nochmals 2 Stunden bei 165 °C gerührt. |

Die so erhaltene wirkstoffhaltige Klebmasse wird mit einer Hotmelt-Beschichtungsanlage (Düsenauftragssystem) so auf eine wiederablösbare Schutzschicht (Hostaphan® RN 100 einseitig mit Silikon beschichtet - Fa. Kalle) beschichtet, daß ein wirkstoffhaltiges Reservoir mit einem Flächengewicht von 80 g/m² resultiert. Auf dieses Reservoir wird die undurchlässige Rückschicht (Polyesterfolie, 15 µm dick) aufkaschiert. Anschließend werden 16 cm² große Wirkstoffpflaster gestanzt.

### Analytik:

- Die Wirkstofffreisetzung der 16 cm² großen transdermalen Pflaster wird nach der in der USP XXII beschriebenen Rotating bottle-Methode in 0,9%iger Kochsalzlösung bei 37 °C bestimmt.
- Zur Messung der Mäusehautpenetration wird die Haut von haarlosen Mäusen in die Franz-Zelle eingespannt. Auf die Haut wird ein estradiolhaltiges Pflaster mit einer Fläche von 2,54 cm² aufgeklebt und die Wirkstofffreisetzung bei 37 °C (Akzeptormedium: 0,9%ige Kochsalzlösung) gemessen (Literatur: Umesh V. Banakar Pharmaceutical dissolution testing (1. Auflage - 1991)).

Die Ergebnisse sind in Tabelle 1 dargestellt.

**Tabelle 1:**

| Analysenergebnisse | | | |
|---|---|---|---|
| Bsp. | Estradiolgehalt µg/16 cm² | in vitro-Freisetzung µg/16 cm² · 4 Std. | Meerschweinchenhautpenetration µg/16 cm² · 24 Std. |
| 1 | 2120 | 367 | 114 |
| nach DE 39 33 460 | 3200 | 1080 | 96 |
| * 48 Std.-Wert minus 24 Std.-Wert | | | |

Wie die Tabelle zeigt, erhält man eine deutlich bessere Penetration durch die Mäusehaut, wie das Vergleichsbeispiel nach DE 39 33 460 unter Beweis stellt.

## Patentansprüche

1. Wirkstoffhaltiges Pflaster zur kontrollierten Abgabe von Estradiol oder seinen pharmazeutisch unbedenklichen Derivaten allein oder in Kombination mit Gestagenen aus einer Rückschicht, einem damit verbundenen wirkstoffhaltigen Reservoir, das unter Verwendung von Haftklebern mit ≥ 6 Gew.% Polymer und 5-94 Gew.% klebrigmachendem Harz und mindestens einem Penetrationsverstärker hergestellt ist und einer ablösbaren Schutzschicht, **dadurch gekennzeichnet, daß** der Penetrationsverstärker aus der Gruppe von Substanzen auf Basis von Carbonsäuren, bestehend aus Glycolsäure, Äpfelsäure, Weinsäure, Mandelsäure, 2-Hydroxyzimtsäure, 3-Hydroxyzimtsäure, trans-4-Methoxyzimtsäure, 2-Hydroxyoctansäure, Tropasäure, Gallussäure, Shikimi-säure, Benzilsäure, Benzol-1,2,4-tricarbonsäure, Dimethyl-3-oxoglutarat, 3-Methyl-2-oxo-valeriansäure, 4-Methyl-2-oxo- valeriansäure, 2-Oxoglutarsäure, Brenztraubensäure, 4-Aminobuttersäure, 6-Aminohexan-säure, 11-Aminoundecansäure, Asparaginsäure, 2-Amino-benzoesäure, 3-Aminobenzoesäure, 4-Aminobenzoesäure, 4-Amino-2- hydroxybenzoesäure, 3-Phenylpropionsäure, 2-Phenylbuttersäure, 4-Phenylbuttersäure, trans-2-Dodecendisäure, Tridecandisäure, Tetradecandisäure, Pentadecandisäure, Diglycolsäure, Piperidin-4-carbonsäure, Pyrazin-2-carbonsäure, Pyrazin-2,3-dicarbonsäure, Pyridin-2-carbonsäure und Nicotinsäure, Pimelinsäuremonomethylester, Malonsäurediamid, Adipinsäurediamid, Pyrazin-2-carbonsäureamid, Bernsteinsäurediamid ausgewählt und in einer Konzentration von 0,01 bis 20 Gew.% im Reservoir enthalten ist.

2. Wirkstoffpflaster nach Anspruch 1, **dadurch gekennzeichnet, daß** das Reservoir Wirkstoff in einer Konzentration von insgesamt 2 - 15 Gew.% enthält.

3. Wirkstoffpflaster nach Anspruch 2, **dadurch gekennzeichnet, daß** bei einer kombinierten Verabreichung das molare Verhältnis von Estradiol oder seinen pharmazeutisch unbedenklichen Derivaten und Gestagenen 1 : 1 bis 1 : 10 ist.

4. Wirkstoffpflaster nach einem der Ansprüche 1 - 3, **dadurch gekennzeichnet, daß** das Reservoir bis zu 5 Gew.-% Hilfsstoffe aus der Gruppe, bestehend aus Farbstoffen, Füllstoffen, Alterungsschutzmitteln und Weichmachern mit Antioxidantien enthält.

5. Wirkstoffpflaster nach einem der Ansprüche 1 - 4, **dadurch gekennzeichnet, daß** das Reservoir aus mehreren Schichten gebildet ist.

6. Wirkstoffpflaster nach einem der Ansprüche 1 - 5, **dadurch gekennzeichnet, daß** die Dicke des Reservoirs im Bereich von 0,02 - 1,0 mm liegt.

7. Wirkstoffpflaster nach einem der Ansprüche 1 - 6, **dadurch gekennzeichnet, daß** das Reservoir im Falle unzureichender Eigenklebrigkeit zur Haut mit einer zusätzlichen haftklebenden Schicht oder einem haftklebenden Rand versehen ist.

8. Verfahren zur Herstellung eines wirkstoffhaltigen Pflasters nach einem der Ansprüche 1 - 7, **dadurch gekennzeichnet, daß** das Reservoir aus Lösung, Dispersion oder Schmelze gebildet wird.

9. Verwendung des Wirkstoffpflasters nach einem der Ansprüche 1 - 8 zur Herstellung eines Arzneimittels für therapeutische Zwecke in der Human- und Veterinärmedizin.

## Claims

1. Active substance-containing patch for the controlled release of estradiol or its pharmaceutically acceptable derivatives alone or in combination with gestagens, comprising a backing layer, an active substance-containing reservoir connected thereto and produced by using pressure-sensitive adhesives with ≥6%-wt. of polymer and 5-94%-wt. of tackifying resin and at least one penetration enhancer, and a removable protective layer, **characterized in that** the penetration enhancer is selected from the group of substances based on carboxylic acids, which group consists of glycollic acid, malic acid, tartaric acid, mandelic acid, 2-hydroxycinnamic acid, 3-hydroxycinnamic acid, trans-4-methoxycinnamic acid, 2-hydroxyoctanoic acid, tropic acid, gallic acid, shikimic acid, benzilic acid, benzene-1,2,4-tricarboxylic acid, dimethyl-3-oxoglutarate, 3-methyl-2-oxo-valerianic acid, 4-methyl-2-oxo-valerianic acid, 2-oxoglutaric acid, pyruviv acid, 4-aminobutyric acid, 6-aminohexanoic acid, 11-aminoundecanoic acid, asparaginic acid, 2-aminobenzoic acid, 3-aminobenzoic acid, 4-aminobenzoic acid, 4-amino-2-hydroxybenzoic acid, 3-phenylpropionic acid, 2-phenylbutyric acid, 4-phenylbutyric acid, trans-2-dodecenedioic acid, tridecanedioic acid, tetradecanedioic acid, pentadecanedioic acid, diglycollic acid, piperidine-4-carboxylic acid, pyrazine-2-carboxylic acid, pyrazine-2,3-dicarboxylic acid, pyridine-2-carboxylic acid and nicotinic acid, pimelic acid monomethyl ester, malonic acid diamide, adipic acid diamide, pyrazine-2-carboxamide, succinic acid diamide, and that the said penetration enhancer is contained in the reservoir in a concentration of 0.01 to 20%-wt.

2. The active substance patch according to claim 1 **characterized in that** the reservoir comprises active substance in the concentration totaling 2 - 15%-wt.

3. The active substance patch according to claim 2 **characterized in that** in case of a combined administration the molar ratio of estradiol or its pharmaceutically acceptable derivatives to gestagens amounts to 1 : 1 to 1 : 10.

4. The active substance patch according to any one of claims 1 to 3 **characterized in that** the reservoir comprises up to 5%-wt. of inactive ingredients of the group consisting of dyes, fillers, anti-ageing agents, and plasticizers with antioxidants.

5. The active substance patch according to any one of claims 1 to 4 **characterized in that** the reservoir is formed of several layers.

6. The active substance patch according to any one of claims 1 to 5 **characterized in that** the thickness of the reservoir is in the range of 0.02 - 1.0 mm.

7. The active substance patch according to any one of claims 1 to 6 **characterized in that** the reservoir, in case it has an insufficient self-tackiness to the skin, is provided with an additional pressure sensitive adhesive layer or with a pressure sensitive adhesive edge.

8. A process for the production of an active substance-containing patch according to any one of claims 1 to 7, **characterized in that** the reservoir is formed from a solution, dispersion, or a melt.

9. The use of the active substance patch according to any one of claims 1 to 8 for the production of a drug for therapeutic purposes in human and veterinary medicine.

## Revendications

1. Emplâtre comprenant un principe actif pour la libération contrôlée d'oestradiol ou de ses dérivés pharmaceutiquement acceptables, à titre individuel ou en combinaison avec des progestatifs, constitué par une couche dorsale, par un réservoir lié à cette dernière contenant un principe actif, qui a été préparé en utilisant des agents auto-adhésifs comprenant un polymère à concurrence de > 6 % en poids et une résine qui rend adhésif à concurrence de 5 à 94 % en poids et au moins un amplificateur de la pénétration, et par une couche de protection pelliculable, **caractérisé en ce que** l'amplificateur de la pénétration est choisi parmi le groupe de substances à base d'acides carboxyliques constitué par l'acide glycolique, l'acide malique, l'acide tartrique, l'acide mandélique, l'acide 2-hydroxycinnamique, l'acide 3-hydroxycinnamique, l'acide trans-4-méthoxycinnamique, l'acide 2-hydroxyoctanoïque, l'acide tropanique, l'acide gallique, l'acide shikimique, l'acide benzilique, l'acide benzène-1,2,4-tricarboxylique, l'acide diméthyl-3-oxoglutarique, l'acide 3-méthyl-2-oxo-valérianique, l'acide 4-méthyl-2-oxo-valérianique, l'acide 2-oxoglutarique, l'acide pyruvique, l'acide 4-aminobutyrique, l'acide 6-aminohexanoïque, l'acide 11-amino-undécanoïque, l'acide aspartique, l'acide 2-aminobenzoïque, l'acide 3-aminobenzoïque, l'acide 4-aminobenzoïque, l'acide 4-amino-2-hydroxybenzoïque, l'acide 3-phénylpropionique, l'acide 2-phénylbutyrique, l'acide 4-phénylbutyrique, l'acide trans-2-dodécanedioïque, l'acide tridécanedioïque, l'acide tétradécanediolque, l'acide pentadécanedioïque, l'acide diglycolique, l'acide pipéridine-4-carboxylique, l'acide pyrazine-2-carboxylique, l'acide pyrazine-2,3-dicarboxylique, l'acide pyridine-2-carboxylique et l'acide nicotinique, l'ester monométhylique de l'acide pimélique, le diamide de l'acide malonique, le diamide de l'acide adipique, l'amide de l'acide pyrazine-2-carboxylique, le diamide de l'acide succinique et est contenu en une concentration de 0,01 à 20 % en poids dans le réservoir.

2. Emplâtre comprenant un principe actif selon la revendication 1, **caractérisé en ce que** le réservoir contient le principe actif en une concentration totale de 2 - 15 % en poids.

3. Emplâtre comprenant un principe actif selon la revendication 2, **caractérisé en ce que**, dans une administration combinée, le rapport molaire de l'oestradiol ou de ses dérivés pharmaceutiquement acceptables et des progestatifs s'élève de 1 : 1 à 1 : 10.

4. Emplâtre comprenant un principe actif selon l'une quelconque des revendications 1 à 3, **caractérisé en ce que** le réservoir contient des adjuvants jusqu'à concurrence de 5 % en poids, choisis parmi le groupe constitué par des colorants, des matières de charge, des agents de protection contre le vieillissement et des plastifiants avec des antioxydants.

5. Emplâtre comprenant un principe actif selon l'une quelconque des revendications 1 à 4, **caractérisé en ce que** le réservoir est formé à l'aide de plusieurs couches.

6. Emplâtre comprenant un principe actif selon l'une quelconque des revendications 1 à 5, **caractérisé en ce que** l'épaisseur du réservoir se situe dans le domaine de 0,02 - 1,0 mm.

7. Emplâtre comprenant un principe actif selon l'une quelconque des revendications 1 à 6, **caractérisé en ce que** le réservoir est muni, dans le cas d'une adhésivité propre insuffisante à la peau, d'une couche auto-adhésive supplémentaire ou d'un bord auto-adhésif.

8. Procédé pour la préparation d'un emplâtre comprenant un principe actif selon l'une quelconque des revendications 1 à 7, **caractérisé en ce qu'**on forme le réservoir en solution, en dispersion ou en fusion.

9. Utilisation de l'emplâtre comprenant un principe actif selon l'une quelconque des revendications 1 à 8, pour la préparation d'un médicament à usage thérapeutique dans la médecine humaine et vétérinaire.
